(19) Europäisches Patentamt
European Patent Office
Office européen des brevets

(11) Numéro de publication : **0 170 583**
**B1**

(12) **FASCICULE DE BREVET EUROPÉEN**

(45) Date de publication du fascicule du brevet : **15.06.88**

(51) Int. Cl.[4] : **C 07 D319/24**, A 61 K 31/335

(21) Numéro de dépôt : **85401420.6**

(22) Date de dépôt : **12.07.85**

(54) **Sels d'ammonium dérivés de hexahydrodibenzodioxane, leurs intermédiaires, leur préparation et leur application en thérapeutique.**

(30) Priorité : **13.07.84 FR 8411174**

(43) Date de publication de la demande : **05.02.86 Bulletin 86/06**

(45) Mention de la délivrance du brevet : **15.06.88 Bulletin 88/24**

(84) Etats contractants désignés : **BE CH DE FR GB IT LI NL SE**

(56) Documents cités :

**Néant**

(73) Titulaire : **RIOM LABORATOIRES- C.E.R.M. "R.L.-CERM" - (S.A.)**
**Route de Marsat B.P. 140**
**F-63203 Riom Cédex (FR)**

(72) Inventeur : **Combourieu, Michel**
**6, rue de Mont Mouchet**
**F-15000 Aurillac (FR)**
Inventeur : **Bernet, Yvon**
**11, Cité des Landes Reilhac**
**F-15250 Jussac (FR)**
Inventeur : **Laigle, Jean-Claude**
**31, Avenue Aristide Briand**
**F-15000 Aurillac (FR)**
Inventeur : **Simbille, Nadine**
**31, Avenue Aristide Briand**
**F-15000 Aurillac (FR)**

(74) Mandataire : **Thérond, Gérard Raymond et al**
**RIOM LABORATOIRES - CERM - S.A. Route de Marsat**
**B.P. 140**
**F-63203 Riom Cédex (FR)**

**EP 0 170 583 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux sels d'ammonium dérivés de 1,2,3,4,4a,10a-hexahydrodibenzo [b,e] [1,4] dioxin-4-ol, les intermédiaires nécessaires à leur préparation et leur application en thérapeutique.

Les nouveaux composés répondent à la formule (I)

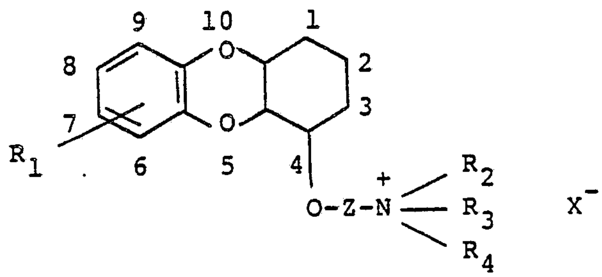

(I)

dans laquelle $R_1$ représente H, un halogène ou un groupe alkoxy ou alkyle de $C_1$ à $C_3$ linéaire ou ramifié, $R_2$, $R_3$ et $R_4$ représentent séparément un groupe alkyle de $C_1$ à $C_4$ linéaire ou ramifié ou $R_2$ ou $R_3$ forment avec l'atome d'azote auquel ils sont liés un radical hétérocyclique saturé, Z représente un groupe alkylène de $C_1$ à $C_6$ linéaire ou ramifié et X représente un anion, de préférence un ion halogène. Par alkyle linéaire ou ramifié de $C_1$ à $C_4$, on entend les groupes méthyle, éthyle, propyle, isopropyle, butyle, pour $R_4$ on préfère le groupe méthyle, pour Z le groupe éthylène, tandis que pour $R_2$ et $R_3$ on préfère les groupes isopropyles.

Par hétérocycle saturé que représente $-NR_2R_3$, on entend les groupes morpholino, pipéridino, pyrrolidino.

Par ion halogénure, on entend $Cl^-$, $Br^-$, $I^-$ ; le bromure est l'ion préféré.

Les composés de formule (I) peuvent être préparés selon l'invention en effectuant les deux réactions suivantes :

a) action sur les alcools de formule (II)

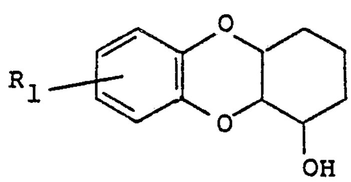

(II)

d'une haloalkylamine de formule

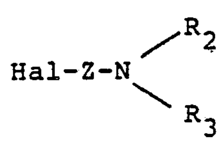

dans laquelle $R_1$, Z, $R_2$ et $R_3$ ont les significations précédentes et Hal représente un halogène, dans des conditions classiques, de préférence en présence d'une base. On peut opérer dans un solvant organique inerte en présence de NaH, t-BuOK ou un alcoolate alcalin, mais on préfère réaliser cette éthérification en présence d'hydroxyde de sodium en solution aqueuse concentrée et d'un catalyseur de transfert de phase sans solvant ou dans un solvant tel que le benzène, ou le toluène.

b) action sur l'amine précédemment obtenue de formule (III)

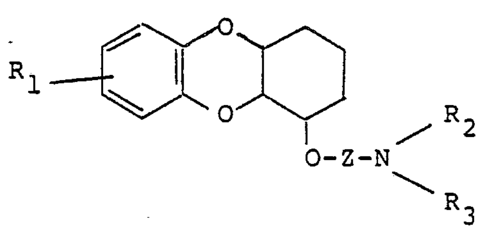

(III)

d'un composé de formule X-$R_4$ dans des conditions classiques pour lequel $R_4$ a la signification précédemment indiquée et X représente le groupe qui forme l'anion $X^-$ après réaction, X étant de préférence un halogène.

Si nécessaire l'anion $X^-$ du composé ainsi obtenu peut être échangé par un autre anion convenable ($X^-$) par des méthodes bien connues dans la littérature.

Les composés de formule (II) sont des composés nouveaux qui sont utiles comme intermédiaire dans la synthèse de différents types de composés ayant notamment une activité thérapeutique.

Ils sont préparés en deux étapes :

a) par réaction sur une ortho-hydroxybenzaldéhyde d'un 3-halocyclohexène en présence d'une base

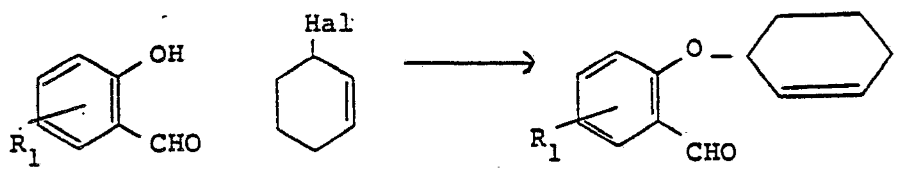

suivie de la cyclisation par

b) action d'un peroxyacide puis d'une base forte sur le composé obtenu avant un traitement par la méthylamine

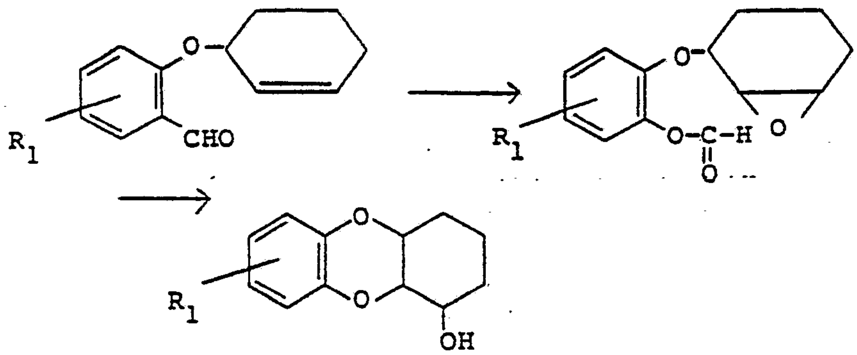

L'étape a) peut être réalisée avec le bromo-3cyclohexène et on opère alors avantageusement en présence de carbonate de potassium, à température ambiante dans l'acétone ; L'étape b) est effectuée par exemple par action de l'acide méta-chloroperbenzoïque dans un solvant chloré comme le dichlorométhane, le milieu réactionnel est ensuite traité par une solution concentrée d'hydroxyde alcalin puis le mélange obtenu après élimination des solvants est traité par la méthylamine en solution alcoolique.

Les ammoniums quaternaires de formule (I) ont des activités pharmacologiques, notamment antispasmodiques, et d'autres objets de l'invention sont les compositions pharmaceutiques comprenant au moins l'un des composés de formule (I) à titre de principe actif. Ces compositions pharmaceutiques seront utiles en thérapeutique humaine notamment dans le traitement des syndromes spasmodiques.

Dans ce qui suit, on décrit des exemples de composés selon l'invention, de leur procédé de synthèse et les résultats obtenus pour certains composés lors d'essais pharmacologiques classiques.

Exemple 1 : 1,2,3,4,4a,10a hexahydro dibenzo [b,e] [1,4] dioxin-4-ol

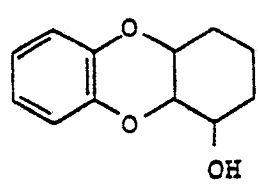

Dans un réacteur de 10 l, on a introduit 633,4 g (5,19 M) d'aldéhyde salicylique, 5 l d'acétone et 717,3 g de carbonate de potassium et laissé sous agitation à température ambiante. Après précipitation du phénate, on a ajouté rapidement goutte à goutte 836 g (5,19 M) de 3-bromocyclohexene, et porté 2 h 1/2 au reflux. Après élimination des sels minéraux par filtration, et évaporation de solvant, on a repris le résidu par de l'éther éthylique, lavé à la soude à 10 % puis à l'eau et ensuite séché la phase éther sur $Na_2SO_4$. Après filtration et élimination de l'éther, on a obtenu 975 g de 1-(2-formyl phenoxy) 2-3 cyclohexene utilisé directement dans l'étape suivante.

Dans une deuxième étape, on a introduit dans un réacteur de 10 l maintenu dans un bain de glace 2 159 g (12,5 M) d'acide méta-chloro-peroxybenzoïque et 5 l de dichlorométhane, puis on a ajouté goutte à goutte 975 g du composé précédent en solution dans 1 l de dichlorométhane et maintenu la température inférieure à 30 °C en laissant la réaction se poursuivre pendant 1 h. On a ensuite filtré l'acide formé et réintroduit le filtrat dans le réacteur ; en maintenant la température inférieure à 30 °C, on a ajouté goutte à goutte 1 000 g de lessive de soude à 50 % (12,5 M). Après avoir laissé une heure sous agitation à température ambiante, on a séparé et lavé la phase organique à l'eau, séché sur $Na_2SO_4$, filtré, évaporé le solvant puis repris le concentrat par 1 l d'éthanol absolu et 0,5 l d'une solution alcoolique à 33 % de méthylamine et porté à reflux pendant 3 h. On a ensuite évaporé l'éthanol, repris par de l'éther éthylique, lavé à l'eau, séché sur $Na_2SO_4$, filtré puis concentré à sec. Par addition de pentane, le composé du titre cristallise. On a ainsi obtenu 587,15 g de produit ayant pour point de fusion F = 81-82 °C et pour analyse élémentaire :

Calculé C 69,88 H 6,84 %
Trouvé C 69,84 H 6,85 %

Exemple 2 : 9-methoxy 1,2,3,4,4a,10a hexahydro dibenzo [b,e] [1,4] dioxin-4-ol

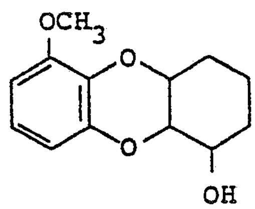

En procédant comme indiqué à l'exemple 1, mais en partant de 64 g de 2-hydroxy 3-methoxy benzaldehyde, on a obtenu 29 g de composé du titre ayant pour point d'ébullition E = 225 °C sous une pression de 6,5 Pa et pour analyse élémentaire :

Calculé C 66,08 H 6,82 %
Trouvé C 65,99 H 6,83 %

Exemple 3 : 4-(2-morpholino) ethoxy 1,2,3,4,4a,10a hexahydro dibenzo [b,e] [1,4] dioxine

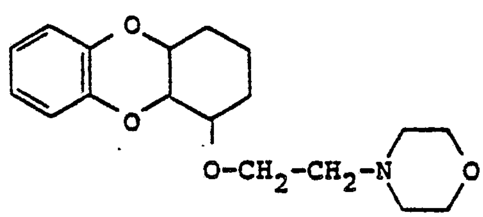

Dans un réacteur de 10 l, on a introduit 6 kg de lessive de soude à 50 %, 1 150 g (5,576 M) de l'alcool de l'exemple 1 et 986 g (5,3 M) de chlorhydrate de 4-(2-chloro) éthyl morpholine et 0,5 g d'éther couronne 18-6 (hexaoxa-1,4,7,10,13,16 cyclooctadécane), puis on a porté le mélange 5 h à 120 °C sous agitation. Après refroidissement, on a extrait à l'éther, lavé à l'eau, séché sur $Na_2SO_4$, filtré, évaporé le solvant et distillé sous vide. On a ainsi obtenu 1 330 g de composé du titre ayant pour point d'ébullition E = 180°C sous une pression de 65 Pa et pour analyse élémentaire :

Calculé C 67,69 H 7,89 N 4,38 %
Trouvé C 67,70 H 8,12 N 4,35 %

Exemple 4 : 4-(2-morpholino) ethoxy-9-methoxy 1,2,3,4,4a,10a hexahydro dibenzo [b,e] [1,4] dioxine

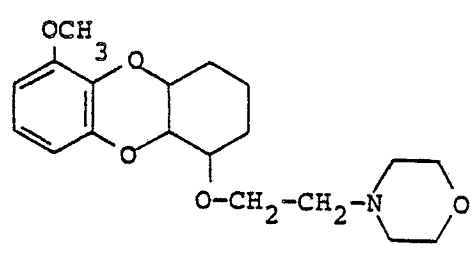

En procédant comme indiqué dans l'exemple 3, mais en partant de 10 g (0,042 M) de l'alcool de l'exemple 2 et de 9 g (0,038 M) de chlorhydrate de 4-(2-chloro) ethyl morpholine, on a obtenu 7,4 g de composé du titre ayant pour point d'ébullition E = 192-195 °C sous une pression de 65 Pa et pour analyse élémentaire :

| | | | |
|---|---|---|---|
| Calculé | C 65,31 | H 7,79 | N 4,01 % |
| Trouvé | C 64,98 | H 7,80 | N 3,97 % |

Exemple 5 : 4-(2-diisopropylamino) ethoxy 1,2,3,4,4a,10a hexahydroxy dibenzo [b,e] [1,4] dioxine

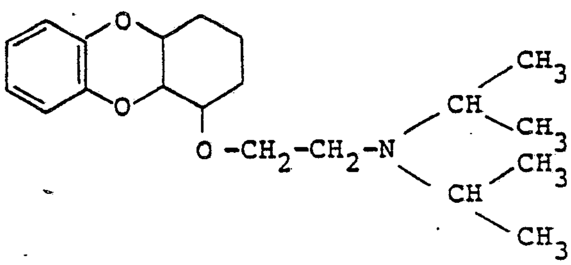

En procédant comme indiqué dans l'exemple 3, mais en partant de 12,4 (0,06 M) de l'alcool de l'exemple 1 et 10 g (0,05 M) de chlorhydrate de 1-chloro-2-diisopropylamino ethane, on a obtenu 13,7 g de composé du titre ayant pour point de fusion F = 63,4 °C et pour analyse élémentaire :

| | | | |
|---|---|---|---|
| Calculé | C 72,03 | H 9,37 | N 4,20 % |
| Trouvé | C 72,04 | H 9,31 | N 4,19 % |

Exemple 6 : 4-(3-dimethylamino) propoxy 1,2,3,4a,10a hexahydro dibenzo [b,e] [1,4] dioxine

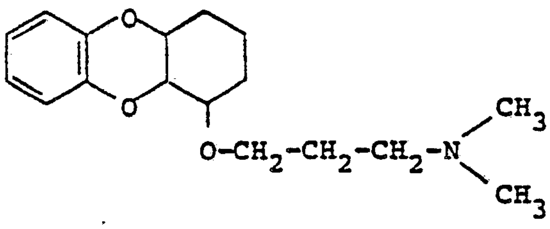

En procédant comme indiqué dans l'exemple 5, mais en partant de 10 g (0,048 M) de l'alcool de l'exemple 1 et de 6,6 g (0,042 M) de chlorhydrate de 3-(dimethylamino)-1-chloro propane, on a obtenu 6,8 g de composé du titre ayant pour point d'ébullition E = 165 °C sous une pression de 1,33 Pa et pour analyse élémentaire :

| | | | |
|---|---|---|---|
| Calculé | C 70,07 | H 8,65 | N 4,81 % |
| Trouvé | C 68,93 | H 8,78 | N 4,76 % |

Exemple 7 : 4-(2,2-dimethyl-3-dimethylamino) propoxy 1,2,3,4,4a,10a hexahydro dibenzo [b,e] [1,4] dioxine

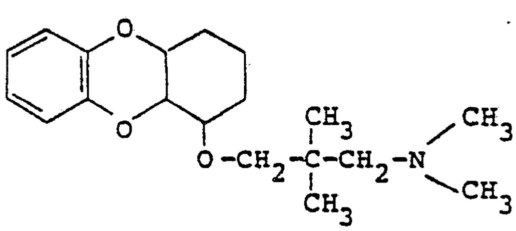

En procédant comme indiqué dans l'exemple 5, mais en partant de 10 g (0,048 M) de l'alcool de l'exemple 1 et de 8,6 g (0,046 M) de chlorhydrate de 1-chloro-2,2-dimethyl-3-dimethylamino propane, on a obtenu 8,6 g de composé du titre ayant pour point d'ébullition E = 135 °C sous une pression de 6,5 Pa et pour analyse élémentaire :

| | | | |
|---|---|---|---|
| Calculé | C 71,44 | H 9,15 | N 4,38 % |
| Trouvé | C 71,57 | H 9,18 | N 4,31 % |

Exemple 8 : Bromure de N-[2-(1,2,3,4,4a,10a hexahydrodibenzo [b,e] [1,4] dioxin 4-yloxy)] ethyl N-methyl

morpholinium

Dans un autoclave, on a dissous 900 g (2,82 M) d'amino-ether de l'exemple 3 dans 3 l d'acétone, puis on a ajouté 535,6 g (5,64 M) de bromure de méthyle. Après début de cristallisation, on a laissé sous agitation pendant 2 h à température ambiante. Après filtration et rinçage à l'acétone, on a remis le solide formé en suspension dans de l'éther de pétrole, agité, filtré, séché sous vide, puis recristallisé dans un minimum du mélange éther diisopropylique/isopropanol (7/3). On a ainsi obtenu 1 000 g de composé du titre ayant pour point de fusion F = 155 °C et pour analyse élémentaire :

Calculé C 55,07 H 6,81 N 3,38 %
Trouvé C 55,40 H 6,80 N 3,37 %

Exemple 9 : Bromure de N-[2-(9-methoxy 1,2,3,4,4a,10a hexahydro dibenzo [b,e] [1,4] dioxin 4-yloxy] ethyl N-methyl morpholinium

Selon le même procédé que celui de l'exemple 8, on est parti de 3,5 g (0,01 M) de l'aminoether de l'exemple 4 pour obtenir après action du bromure de méthyle 3,3 g de produit du titre ayant pour point de fusion F = 167,1 °C et pour analyse élémentaire :

Calculé C 54,06 H 6,81 N 3,15 %
Trouvé C 53,89 H 6,90 N 3,13 %

Exemple 10 : Bromure de N-[(2-(1,2,3,4,4a,10a hexahydrodibenzo [b,e] [1,4] dioxin 4-yloxy)] ethyl N-methyl diisopropyl ammonium

Selon le même procédé que celui de l'exemple 8, partant de 5,6 g (0,017 M) de l'aminoether de l'exemple 5, on a obtenu 4,5 g de produit du titre ayant pour point de fusion F = 155,5 °C et pour analyse élémentaire :

Calculé C 58,87 H 8,00 N 3,27 %
Trouvé C 58,06 H 8,15 N 3,21 %

Exemple 11 : Iodure de N-[2-(1,2,3,4,4a,10a hexahydrodibenzo [b,e] [1,4] dioxin-4-yloxy)-ethyl]-N-methyl morpholinium

Selon le même procédé que celui de l'exemple 8, mais en partant de 600 g (1,88 M) du composé de l'exemple 3 et de 537,4 g (3,76 M) de l'iodure de méthyle, on a obtenu 561 g de composé du titre ayant pour point de fusion F = 162 °C.

Les ammonium quaternaires de formule (I) se sont révélés posséder d'intéressantes propriétés antispasmodiques mises en évidence in vitro selon les méthodes usuelles de pharmacologie moléculaire. Pour l'activité anticholinergique, on a utilisé comme agoniste l'acétylcholine et comme effecteur biologique le jéjunum de rat. Pour l'activité antihistaminique et pour l'activité inhibitrice de la perméabilité membranaire induite par $BaCl_2$, on a utilisé respectivement l'histamine et $BaCl_2$, et comme effecteur biologique l'iléon de cobaye. Les $pA_2$ caractéristiques des substances étudiées, calculés selon la méthode de Van Rossum [Arch. Int. Pharmacodyn. 143, 299-330 (1963)] sont rapportés dans le tableau ci-après :

| Composés n° | Activité anticholinergique | Activité antihistaminique | Inhibition perméabilité membranaire |
|---|---|---|---|
| 8 | 5,45 ± 0,151 | 5,5 ± 0,24 | 5,0 ± 0,22 |
| 9 | 4,6 ± 0,10 | Inactif à $10^{-4}$ M | 4,1 (à $10^{-4}$ M seulement) |
| 10 | 6,1 ± 0,12 | 4,1 ± 0,11 | 5,3 ± 0,35 |

Ces résultats montrent que les composés de l'invention possèdent d'intéressantes propriétés antispasmodiques, le composé n° 8 ayant des niveaux d'activité comparables vis-à-vis des trois agonistes utilisés. Ces composés présentent en outre une très faible toxicité. A 640 mg/kg, voie orale, chez la souris,

on n'a relevé aucune mortalité : la DL 50 peut donc être estimée ⩾ 2 g/kg per os. On peut donc envisager leur utilisation en thérapeutique humaine dans le traitement des manifestations spasmodiques de toutes origines.

Associés aux excipients pharmaceutiques habituels, ils peuvent être administrés par voie enthérale ou parenthérale à une dose journalière comprise entre 0,1 mg et 15 mg par kg de poids corporel selon les voies d'administration. En thérapeutique humaine, on peut utiliser une dose journalière comprise entre 10 mg et 1 000 mg, sachant que l'administration par voie orale est préférée.

Mélangés avec des excipients convenables, les composés de formule I ou un de leurs sels peuvent être comprimés sous forme de prise unitaire tels que comprimés, pilules etc. ou peuvent être mis en gélules.

Au moyen de liquides convenables, les composés de formule I peuvent aussi être utilisés en préparation injectable ou orale sous forme de solutions, suspensions ou émulsions.

Les composés de formule I possèdent un carbone asymétrique de sorte qu'il est possible d'obtenir un mélange racémique de I et les énantiomères optiques séparés. Le mélange racémique et les énantiomères optiques séparés appartiennent à la fois aux composés de l'invention.

Les énantiomères optiques peuvent être obtenus de manière habituelle par résolution du mélange racémique, ou directement en partant de matières premières optiquement actives.

**Revendications**

1. Composés répondant à la formule I

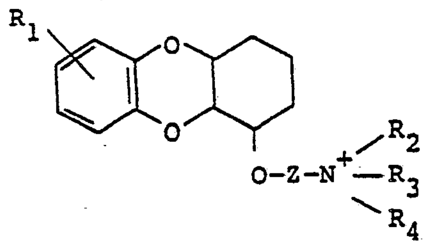

$X^-$

(I)

dans laquelle $R_1$ représente H, un halogène ou un groupe alkoxy ou alkyle de $C_1$ à $C_3$ linéaire ou ramifié, $R_2$, $R_3$ et $R_4$ représentent séparément un groupe alkyle de $C_1$ à $C_4$ linéaire ou ramifié ou $R_2$ et $R_3$ forment avec l'atome d'azote auquel ils sont liés un radical hétérocyclique saturé, Z représente un groupe alkylène de $C_1$ à $C_6$ linéaire ou ramifié et $X^-$ représente un anion, de préférence un ion halogène.

2. Composés selon la revendication 1 pour lesquels Z représente $-CH_2-CH_2-$

3. Composés selon la revendication 1 pour lesquels $R_2$ et $R_3$ représentent l'isopropyle.

4. Composés selon la revendication 1 pour lesquels

$$-N{<}^{R_2}_{R_3}$$

représente le radical morpholino.

5. Composés selon la revendication 1 pour lesquels $R_4$ représente le méthyle.

6. Composés selon la revendication 1 pour lesquels $X^-$ représente $Br^-$.

7. Composés répondant à la formule II

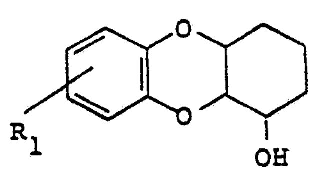

(II)

dans laquelle $R_1$ a la signification indiquée dans la revendication 1.

8. Composés répondant à la formule III

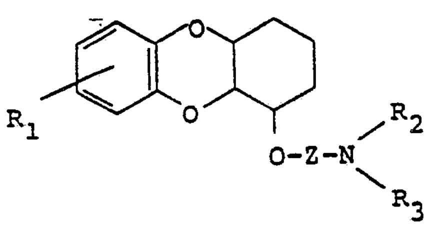

(III)

dans laquelle $R_1$, Z, $R_2$, $R_3$ ont les significations données dans la revendication 1.

9. Composition pharmaceutique à activité antispasmodique caractérisée en ce qu'elle contient au moins un des composés selon la revendication 1 associé avec un ou plusieurs excipients pharmaceutiques habituels.

10. Composition pharmaceutique selon la revendication 9, caractérisée en ce qu'elle contient, à titre de principe actif, le composé de formule

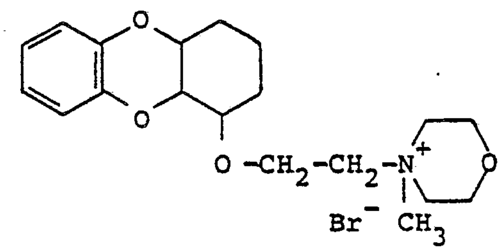

11. Procédé de préparation des composés selon la revendication 1, caractérisé en ce qu'on fait réagir un composé de formule X-$R_4$ avec une amine de formule III

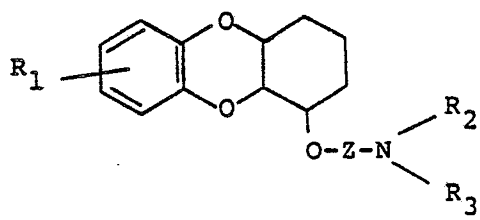

(III)

dans lesquels $R_1$, $R_2$, $R_3$, $R_4$ et Z ont les significations indiquées dans la revendication 1 et X représente le groupe qui forme l'anion $X^-$ après réaction, X étant de préférence un halogène.

12. Procédé pour la préparation d'un composé de formule III selon la revendication 8, caractérisé en ce qu'on fait réagir de préférence en présence d'une base un alcool de formule II

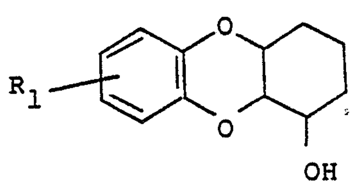

(II)

avec un haloalkylamine de formule

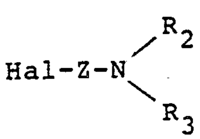

dans lesquelles Z, $R_1$, $R_2$, $R_3$ ont les significations indiquées dans la revendication 1 et Hal représente un halogène.

13. Procédé pour la préparation d'un composé de formule II

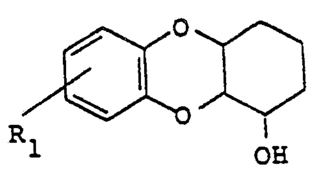

(II)

dans laquelle $R_1$ a la signification indiquée dans la revendication 1 caractérisé en ce que

a) on fait agir une ortho-hydroxybenzaldéhyde sur un 3-halocyclohexène en milieu basique,
b) on traite le composé ainsi obtenu par un péroxyacide,
c) on traite le composé ainsi obtenu par une base forte, puis par la méthylamine.

**Claims**

1. Compounds of the formula I

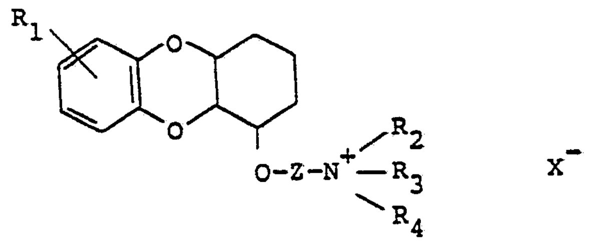

(I)

in which $R_1$ denotes H, halogen or a linear or branched $C_1$ to $C_3$ alkoxy or alkyl group, $R_2$, $R_3$ and $R_4$ separately denote a linear or branched $C_1$ to $C_4$ alkyl group or $R_2$ and $R_3$4 together with the nitrogen atom to which they are bound form a saturated heterocyclic radical, Z denotes a linear or branched $C_1$ to $C_6$ alkylene group and $X^-$ denotes an anion, preferably a halogen ion.

2. Compounds according to claim 1 in which Z denotes $-CH_2-CH_2-$.

3. Compounds according to claim 1 in which $R_2$ and $R_3$ denote isopropyl.

4. Compounds according to claim 1 in which

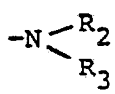

denotes the morpholino radical.

5. Compounds according to claim 1 in which $R_4$ denotes methyl.

6. Compounds according to claim 1 in which $X^-$ denotes $Br^-$.

7. Compounds of the formula II

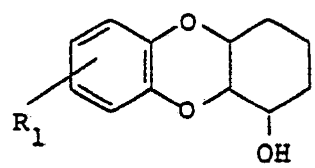

(II)

in which $R_1$ has the meaning defined in claim 1.

8. Compounds of the formula III

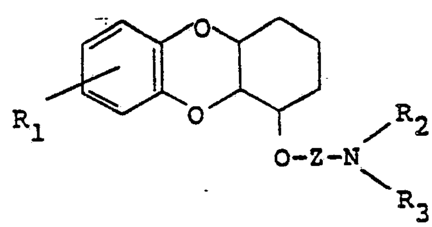

(III)

in which $R_1$, Z, $R_2$ and $R_3$ have the meanings defined in claim 1.

9. Pharmaceutical composition for antispasmodic activity, characterised in that it contains at least one of the compounds according to claim 1, together with one or more usual pharmaceutical auxiliaries.

10. Pharmaceutical composition according to claim 9, characterised in that it contains, as the active principle, the compound of formula

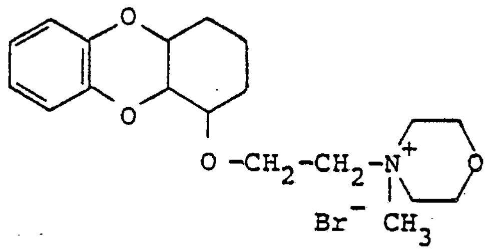

11. Process for the preparation of the compounds according to claim 1, characterised in that a compound of the formula X-$R_4$ is reacted with an amine of formula III

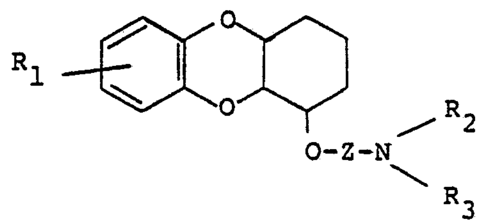

(III)

in which $R_1$, $R_2$, $R_3$, $R_4$ and Z have the same meaning as defined in claim 1 and X represents the group that forms the anion $X^-$ after the reaction, X preferably being halogen.

12. Process for the preparation of a compound of the formula III according to claim 8, characterized in that an alcohol of formula II

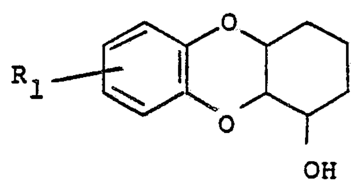

(II)

is reacted, preferably in the presence of a base, with a halo alkylamine of the formula

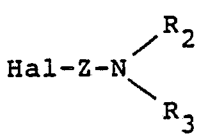

in which Z, $R_1$, $R_2$ and $R_3$ have the significance defined in claim 1 and Hal represents halogen.

13. Process for the preparation of a compound of formula II

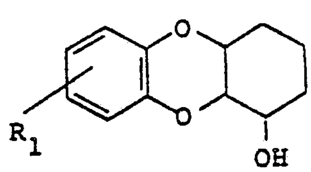

(II)

in which $R_1$ has the meaning defined in claim 1, characterized in that

(a) 3-halocyclohexene is reacted with an orthohydroxybenzaldehyde in the presence of a base,

(b) the compound thus obtained is reacted with a per(oxy) acid, after which

(c) the compound thus obtained is subsequently treated with a strong base and then with methylamine.

**Patentansprüche**

1. Verbindungen der Formel I

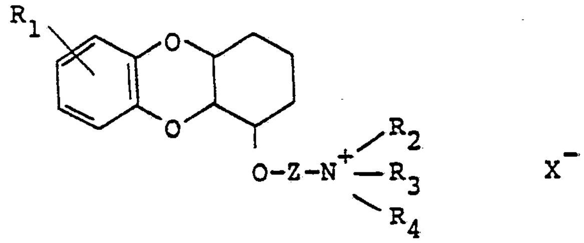

(I)

worin $R_1$ für H, ein Halogen oder eine lineare oder verzweigte $C_1$-$C_3$-Alkoxy- oder -Alkylgruppe und $R_2$, $R_3$ und $R_4$ unabhängig für eine lineare oder verzweigte $C_1$-$C_4$-Alkylgruppe stehen oder $R_2$ und $R_3$ zusammen mit dem Stickstoffatom, an das sie gebunden sind, einen gesättigten heterocyclischen Rest bilden, sowie Z für eine lineare oder verzweigte $C_1$-$C_6$-Alkylengruppe und $X^-$ für ein Anion, vorzugsweise ein Halogenion, stehen.

2. Verbindungen nach Anspruch 1, worin Z für $-CH_2-CH_2-$ steht.

3. Verbindungen nach Anspruch 1, worin $R_2$ und $R_3$ für Isopropyl stehen.

4. Verbindungen nach Anspruch 1, worin

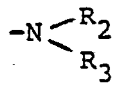

den Morpholinrest darstellt.

5. Verbindungen nach Anspruch 1, worin $R_4$ für Methyl steht.

6. Verbindungen nach Anspruch 1, worin $X^-$ für $Br^-$ steht.

7. Verbindungen der Formel II

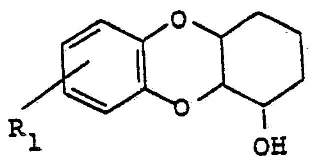

(II)

worin $R_1$ die in Anspruch 1 angegebene Bedeutung hat.

8. Verbindungen der Formel III

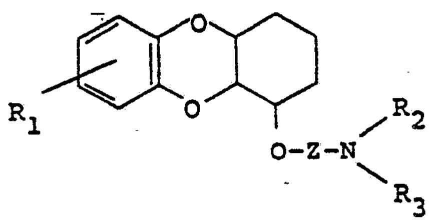

(III)

worin $R_1$, Z, $R_2$ und $R_3$ die in Anspruch 1 angegebenen Bedeutungen haben.

9. Pharmazeutische Zusammensetzung mit spasmolytischer Wirkung, dadurch gekennzeichnet, dass sie mindestens eine der Verbindungen nach Anspruch 1 zusammen mit einem oder mehreren üblichen pharmazeutischen Trägerstoffen enthält.

10. Pharmazeutische Zusammensetzung nach Anspruch 9, dadurch gekennzeichnet, dass sie als Wirkstoff die Verbindung der Formel

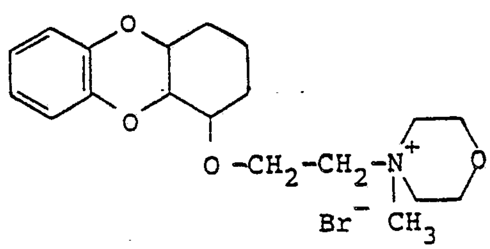

enthält.

11. Verfahren zur Herstellung von Verbindungen nach Anspruch 1, dadurch gekennzeichnet, dass man eine Verbindung der Formel X-$R_4$ mit einem Amin der Formel III

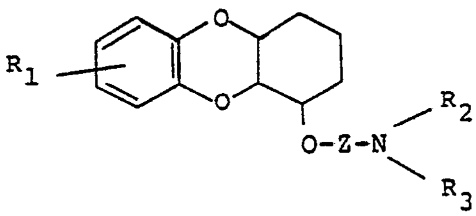

(III)

worin $R_1$, $R_2$, $R_3$, $R_4$ und Z die Anspruch 1 angegebenen Bedeutungen haben und X für die Gruppe steht, welche nach der Reaktion das Anion $X^-$ bildet, wobei X vorzugsweise ein Halogen ist, zur Reaktion bringt.

12. Verfahren zur Herstellung einer Verbindung der Formel III nach Anspruch 8, dadurch gekennzeichnet, dass man einen Alkohol der Formel II

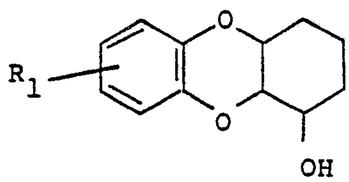

(II)

vorzugsweise in Gegenwart einer Base mit einem Halogenalkylamin der Formel

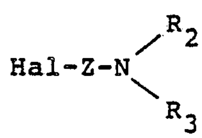

worin Z, $R_1$, $R_2$ und $R_3$ die in Anspruch 1 angegebenen Bedeutungen haben und Hal für ein Halogen steht, zur Reaktion bringt.

13. Verfahren zur Herstellung einer Verbindung der Formel II

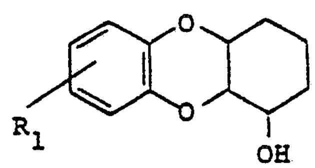

(II)

worin $R_1$ die in Anspruch 1 angegebene Bedeutung hat, dadurch gekennzeichnet, dass man

a) einen ortho-Hydroxybenzaldehyd in basischem Milieu auf ein 3-Halogencyclohexen einwirken lässt,

b) die so erhaltene Verbindung mit einer Peroxysäure und

c) die so erhaltene Verbindung mit einer starken Base und dann mit Methylamin behandelt.